Europäisches Patentamt

(19) European Patent Office

Office européen des brevets

(11) Publication number: **0 256 856**
**A2**

## (12) EUROPEAN PATENT APPLICATION

(21) Application number: **87307152.6**

(22) Date of filing: **13.08.87**

(51) Int. Cl.³: **A 61 K 9/10**
**A 61 K 9/50**

(30) Priority: **14.08.86 US 896844**

(43) Date of publication of application:
**24.02.88 Bulletin 88/8**

(84) Designated Contracting States:
**AT BE CH DE ES FR GB GR IT LI LU NL SE**

(71) Applicant: NEOMED CORPORATION
540 Hunter Court
Wilmette Illinois 60091(US)

(72) Inventor: Ecanow, Bernard
540 Hunter Court
Wilmette Illinois 60091(US)

(74) Representative: Goodenough, Nigel et al,
A.A. Thornton & Co. Northumberland House 303-306 High
Holborn
London WC1V 7LE(GB)

(54) A parenterally administrable composition.

(57) A parenterally administrable composition is based upon
a two phase liquid aqueous coacervate system useful for the
solubilization and delivery of a parent compound or salt
thereof of a drug which is usually parenterally administered.
The composition including a medicament demonstrates
normal pharmacokinetics and can be administered at the pH
of body tissue. The invention also provides a method for pre-
paring the composition whereby sterile, effective solutions of
injectable drugs can be provided.

EP 0 256 856 A2

- 1 -

## A PARENTERALLY ADMINISTRABLE COMPOSITION

This invention concerns a parenterally administrable composition which enables the solubilization and delivery of stable drug components in parenteral form at the pH of body tissue. More particularly the invention provides a drug delivery system which allows for rapid dissolution and smooth liberation of the drug without precipitation, and further provides a convenient method for preparing the composition.

The term "drug" is defined by the Federal Food, Drug and Cosmetic Act, and as used in the present disclosure, as "articles recognized in the official United States Pharmacopoeaia, official Homeopathic Pharmacopoeaia, or official National Formulary or any supplement to any of them." Further clarification of this definition is supported by Remington's Pharmaceutical Sciences, which states "drug to mean any article contained in the above-cited references which is used in the process of diagnosis, cure, treatment, mitigation or prevention of disease in man and animals." Remington's Pharmaceutical Sciences, (Easton, Pa.: Mack Publishing Co., 1975), p. 1843.

It is known that the active component of many parenteral drugs such as antibiotics, anesthetics, anticancer agents, hypertensive compounds, etc., are insoluble or only slightly soluble at the pH of plasma or tissue fluids and, as such, the procedures commonly employed to deal with this problem include use of the salt of the parent compound or use of propylene glycol and alcohol in an alkaline or an acidic medium as a solubilizing agent for the drug. Physician's Desk Reference, (Oradell, N.J.: Medical Economic Co., 1986).

U.S. Pharmacopoeaia indicates that of the injectable drugs used in the direct medical treatment of diseased states, approximately 200 are prepared from the salt of the parent compound and administered at a pH which differs significantly from that of body tissue. It follows from accepted pharmaceutic principles that when a drug based upon the salt of a parent compound is injected, the intra and extracellular water at the site of injection will dilute the drug sufficiently to alter its pH and cause a quantity of it to precipitate out of solution and into the injected tissue. Until the precipitate is dissolved by body fluids, full effectiveness of the drug is compromised. Dilution, alteration of the pH and precipitation cause the rate of drug delivery to become erratic with consequent delay and adverse effects on absorption and effectiveness. In instances where a drug, for example, lidocaine HCl having a pH of from 3.5 to 5.5, is used to control cardiac arryhthmias, any delay in absorption due to a precipitate forming at the site of injection may prove to be life threatening.

Additionally, in drugs of the variety referred to above, an incompatible pH and the deposit of drug precipitate into the injected site contributes significantly to the intense, localized pain and tissue damage associated with the administration of many parenteral drugs. Whereas anti-cancer drugs are notable for their excessive pH and significant damage to the site of injection upon parenteral administration, many other drugs with similar characteristics, like methohexital sodium and acetozolamide, are also known to cause severe pain and other deleterious effects with injection.

The following list illustrates the extensive use of the salt of the parent compound in injectable drugs as well as the frequency of an associated excessive pH. As is evident, virtually all classes of drugs are represented, and are suitable for formulation in the disclosed invention.

| DRUG | pH |
|---|---|
| Acetazolamide, Sodium | 9.0-10.0 |
| Alphaprodine HCl | 4.0-6.0 |
| Aminocaproic Acid | 6.0-7.6 |
| Aminosuppurate Sodium | 6.7-7.6 |
| Aminophylline | 8.6-9.0 |
| Aminotryptyline HCl | 4.0-6.0 |
| Amobarbitol Sodium | 9.6.10.4 |
| Anileridine | 2.5-3.0 |
| Amphotericin B | 7.2-8.0 |
| Ampicillin | 5.0-7.0 |
| Anti coagulant Heparin Solution | 5.0-7.0 |
| Arginine HCl | 5.0-6.5 |
| Atropine Sulfate | 3.0-6.5 |

| | |
|---|---|
| Azathioprine Sodium | 9.8-11.0 |
| Benztropine Mesylate | 5.0-8.0 |
| Betaine HCl | 0.80-1.2 |
| Betamathazone Sodium | 8.0 |
| Bethanecol Chloride | 5.5-7.5 |
| Biperiden Lactate | 4.8-5.8 |
| Bleomycin Sulfate | 4.5-6.0 |
| Brompheniramine Maleate | 6.8-7.0 |
| Bupivacaine-Epinephrine Injection | 3.3-8.5 |
| Bupivacaine HCl | 4.0-6.5 |
| Butabarbitol Sodium | 10.0-11.2 |
| Butorphanol Tartrate | 3.0-5.5 |
| Caffeine-Sodium Benzoate Injection | 6.5-8.5 |
| Calcium Glueptate Injection | 5.6-7.0 |
| Calcium Levulinate | 6.0-8.0 |
| Carboprost Tromethiamine Injection | 7.0-8.0 |
| Cefamandole Sodium | 6.0-8.5 |
| Cefamandole Nafate | 6.0-8.0 |
| Cephazolin Sodium | 4.5-7.0 |
| Cefataxime Sodium | 4.5-6.5 |
| Ceftizoxime Sodium | 4.5-65. |
| Cephalothin Sodium | 4.5-8.0 |
| Cephaprin Sodium | 6.5-8.0 |
| Cephradine | 8.0-9.6 |
| Cefonocid Sodium | |
| Chloramphenicol | 5.0-8.0 |
| Chlordiazepoxide HCl | 2.5-3.5 |
| Chloroprocaine HCl | 2.7-4.0 |
| Chlorothiazide Sodium | 9.2-10.0 |
| Chlorpromazine HCl | 3.0-5.0 |
| Cefoperazone Sodium | 4.5-6.5 |
| Chlorphenramine Maleate | 4.0-5.2 |
| Chloroquine HCl | 5.5-6.5 |
| Chlortetracycline NCl | 2.3-3.3 |
| Clorprothixene | 4.0-5.0 |
| Colchicine Desmopressin | 6.0-7.0 |
| Clindamycin Phosphate | 5.5-7.7 |
| Cimetadine Hydrochloride | 4.0-6.0 |
| Codeine Phosphate | 3.0-6.0 |
| Corticotropin | 2.5-6.0 |
| Cyanocobalamin | 4.5-7.0 |
| Cyclizine Lactate | 3.2-4.7 |
| Cyclophosphamide | 3.9-6.7 |
| Cyclosporine | |
| Cysteine HCl | 1.0-2.5 |
| Chlorprothixene HCl | 3.4 |
| Dantrolene Sodium | 9.5 |
| Dacarbazine | 3.0-4.0 |
| Dactinomycin | 5.5-7.5 |
| Daunorubicin HCl | 4.5-6.5 |
| Deslanoside | 5.5-7.0 |
| Desmopressin Acetate | 3.0-5.0 |
| Dexamethasone Sodium Phosphate | 7.0-8.5 |
| Diatrizoate Meglumine | 6.0-7.7 |
| Diatrizoate Sodium | 4.5-7.5 |
| Diazepam | 6.2-6.9 |
| Diazoxide | 11.2-11.9 |
| Dibucaine HCl | 4.5-7.0 |

| | |
|---|---|
| Dicyclomine HCl | 5.0-5.5 |
| Ditheylstilbesterol Disphosphate | |
| Digoxin | |
| Dihydroergotamine Mesylate | 3.2-4.0 |
| Diphenhydramine HCl | 4.0-6.5 |
| Dimenhydrinate | 6.4-7.2 |
| Dobutamine HCl | 2.5-5.5 |
| Dopamine HCl | 2.5-5.5 |
| Dopamine HCl -Dextrose | 3.0-5.0 |
| Doxapram HCl | 3.5-5.0 |
| Doxorubicin HCl | 3.8-6.5 |
| Droperidol | 3.0-3.8 |
| Dyphylline | 5.0-8.0 |
| Edetate Disodium | 6.5-7.5 |
| Emetine HCl | 3.0-4.0 |
| Ephedrine Sulfate | 4.5-7.0 |
| Epinephrine | 2.5-5.0 |
| Ergonovine Maleate | 2.7-3.5 |
| Ergotamine Tartrate | 3.0-4.0 |
| Erythromycin | |
| Erythromycin Ethylsuccinate | 6.0-8.5 |
| Erythromycin Gluceptate | 6.0-8.0 |
| Erythromycin Lactibionate | 6.5-7.5 |
| Estradiol Valerate | |
| Ethacrynate Sodium | 6.3-7.7 |
| Ethylnorepinephrine HCl | 2.5.5.0 |
| Etidocaine HCl | 11.0 |
| Fentanyl Citrate | 4.0-7.5 |
| Floxuridine | 4.0-5.5 |
| Fluorescein Sodium | 8.0-9.0 |
| Fluoracil | 8.6-9.0 |
| Fluphenazine Enanthate | |
| Fluphenazine HCl | 4.8-5.2 |
| Folic Acid | 8.0-11.0 |
| Furosemide | 8.5-9.3 |
| Gallamine Triethiodide | 5.3-7.0 |
| Gentamycin Sulfate | 3.0-5.5 |
| Glucagon | 2.5-3.0 |
| Glycopyrrolate | 2.0-3.0 |
| Haloperidol | 3.0-3.8 |
| Heparin-Calcium | 5.0-7.5 |
| Heparin-Sodium | 5.0-7.5 |
| Hetacillin-Potassium | 7.0-9.0 |
| Hexafluorenium Bromide | 4.0-7.0 |
| Histamine Phosphate | 3.0-6.0 |
| Hyaluranidase | 6.4-7.4 |
| Digitoxin | |
| Fructose | 3.0-6.0 |
| Hydralazine HCl | 3.4-4.4 |
| Hydrocortisone Sodium Phosphate | 7.5-8.5 |
| Hydrocortisone Sodium Succinate | 7.0-8.0 |
| Hydromorphone HCl | 4.0-5.0 |
| Hydoxocobalamin | 3.5-5.0 |
| Hydroxyzine HCl | 3.5-6.0 |
| Hyoscyamine Sulfate | 3.0-4.5 |
| Imipramine HCl | 4.0-5.0 |
| Iophendylate | 6.5-7.7 |
| Iothalamate Sodium | 6.5-7.7 |
| Iron Dextran | 5.2-6.5 |

| | |
|---|---|
| Isobucaine HCl-Epinephrine | |
| Isoniazid | 6.0-7.0 |
| Isoproterenol HCl | 3.5-4.5 |
| Isoxsuprine HCl | 4.9-6.0 |
| Kanamycin Sulfate | 3.5-5.0 |
| Ketamine HCl | 3.5-4.5 |
| Leucovorin Calcium | 6.5-8.5 |
| Levallorphan Tartrate | 4.0-4.5 |
| Levorphanol Tartrate | 4.1-4.5 |
| Lidocaine HCl | 5.0-7.0 |
| Lidocaine HCl Dextrose | 3.5-7.0 |
| Lidocaine HCl-Epinephrine | 3.3-5.5 |
| Lidocaine HCl-Epinephrine Bitartrate | 3.3-5.5 |
| Lincomycin HCl | 3.0-5.5 |
| Magnesium Sulfate | 5.5-7.0 |
| Magnesium Chloride | 1.5-2.5 |
| Mechlorethamine HCl | 3.0-5.0 |
| Menotropins | 6.0-7.0 |
| Meperidine HCl | 3.5-6.0 |
| Mephentermine Sulfate | 4.0-6.5 |
| Mepivacaine HCl | 4.5-6.8 |
| Mepivacaine HCl-Levonordefrin | 3.3-5.5 |
| Meprylcaine HCl-Epinephrine | 3.5-5.5 |
| Mesoridazine Besylate | 4.0-5.0 |
| Metaraminol Bitartrate | 3.2-4.5 |
| Methadone HCl | 3.0-6.5 |
| Methicillin Sodium | 5.0-7.5 |
| Methiodal Sodium | 5.0-8.0 |
| Methocarbamol | 3.5-6.0 |
| Methohexital Sodium | 10.6-11.6 |
| Methotrexate Sodium | 8.0-9.0 |
| Methotrimeprazine | 3.0-5.0 |
| Methoxamine HCl | 3.0-5.0 |
| Methscopolamine Bromide | 4.5-6.0 |
| Methyldopate HCl | 3.0-4.2 |
| Methylergonovine Maleate | 2.7-3.5 |
| Methylpredisolone Sodium Succinate | 7.0-8.0 |
| Metronidazole | 4.5-7.0 |
| Miconazole | 3.7-5.7 |
| Minocycline HCl | 2.0-3.5 |
| Mitomycin | 6.0-8.0 |
| Morphine Sulfate | 2.5-6.0 |
| Moxalactam Disodium | 4.5-7.0 |
| Nafcillin Sodium | 6.0-8.5 |
| Naloxone HCl | 3.0-4.5 |
| Neostigmine Methylsulfate | 5.0-6.5 |
| Netilmicin Sulfate | 3.5-6.0 |
| Niacin | 4.0-6.0 |
| Niacinamide | 5.0-7.0 |
| Norepinephrine Bitartrate | 3.0-4.5 |
| Nylidrin HCl | 4.5-6.5 |
| Orphenadrine Citrate | 5.0-6.0 |
| Oxacillin Sodium | 5.0-8.5 |
| Oxymorphone HCl | 2.7-4.5 |
| Oxytetracycline | 8.0-9.0 |
| Oxytetracycline HCl | 2.0-3.0 |

| | |
|---|---|
| Oxytocin | 2.5-4.5 |
| Papaverine HCl | 3.0 or less |
| Parathyroid | 2.5-3.5 |
| Penicillin G Potassium | 6.5-8.5 |
| Penicillin G Procaine | 5.0-7.5 |
| Penicillin G Sodium | 6.5-7.5 |
| Pentazocine Lactate | 4.0-5.0 |
| Phenobarbital Sodium | 9.0-10.5 |
| Perphenazine | 4.2-5.6 |
| Phenobarbital Sodium | 9.2-10.2 |
| Phentolamine Mesylate | 4.5-6.5 |
| Phenylephrine HCl | 3.0-6.5 |
| Phenytoin Sodium | 10.0-12.3 |
| Physostigmine Salicylate | 4.0-6.0 |
| Phytonadione | 3.5-7.0 |
| Plicamycin | 5.0-7.5 |
| Posterior Pituitary | 2.5-4.5 |
| Potassium Acetate | 5.5-8.0 |
| Potassium Chloride | 4.0-8.0 |
| Prednisolone Sodium Phosphate | 7.0-8.0 |
| Prednisolone Sodium Succinate | 6.7-8.0 |
| Prilocaine HCl | 6.0-7.0 |
| Procainamide HCl | 4.0-6.0 |
| Procaine HCl | 3.0-5.5 |
| Procaine HCl-Epinephrine | 3.0-5.5 |
| Procaine-Phenylephrine Hydrochlorides | 3.0-5.5 |
| Procaine and Tetracaine HCl and Levonodefrin | 3.5-5.0 |
| Prochlorperazine Edisylate | 4.2-6.2 |
| Promazine HCl | 4.0-5.5 |
| Promethazine HCl | 4.0-5.5 |
| Propiomazine HCl | 4.7-5.3 |
| Propoxycaine-Procaine HCl's-Norepinephrine Bitartrate | 3.5-5.0 |
| Propanolol HCl | 2.8-4.0 |
| Protein Hydrolysate | 4.0-7.0 |
| Pyridostigmine Bromide | 4.5-5.5 |
| Pyridoxine HCl | 2.0-3.8 |
| Quinidine Gluconate | 3.0-4.0 |
| Reserpine | 4.5-7.0 |
| Riboflavin | 4.8-5.5 |
| Ritodrine HCl | 3.0-4.5 |
| Rolitetracycline | 3.5-6.5 |
| Scopolamine HCl | 9.0-10.5 |
| Secobarbital Sodium | 2.5-5.5 |
| Sisomycin Sulfate | 3.8-5.6 |
| Spectinomycin HCl | 5.0-8.0 |
| Streptomycin Sulfate | 3.0-4.5 |
| Succinylcholine Chloride | 8.5-10.5 |
| Sulfadizazine Sodium | 7.0-8.5 |
| Sulfisoxazole Diolamine | 3.0-5.0 |
| Terbutaline Sulfate | |
| Testosterone Cypionate | |
| Testosterone Enanthate | |
| Tetracaine HCl | 3.2-6.0 |

| | |
|---|---|
| Tetracycline HCl | 2.0-3.0 |
| Tetracycline Phosphate Complex | 2.0-3.0 |
| Thiamine HCl | 2.5-4.5 |
| Thiamylal Sodium | 10.7-11.5 |
| Thiethylperazine Maleate | 3.0-4.0 |
| Thiopental Sodium | 10.2-11.2 |
| Thiothixene HCl | 2.5-3.5 |
| Tobramycin Sulfate | 6.0-8.0 |
| Tolazoline HCl | 3.0-4.0 |
| Tolbutaminde Sodium | 8.0-9.0 |
| Triamcinolane Diacetate | 6.0 |
| Tridihexethyl Chloride | 5.0-7.0 |
| Trifluoperazine HCl | 4.0-5.0 |
| Triflupromazine HCl | 3.5-5.2 |
| Trimethaphan Camsylate | 4.9-5.6 |
| Trimethobenzamide HCl | 4.8-5.2 |
| Trimethoprimsulfamethoxazole | 10.0 |
| Tromethamine | 10.0-11.5 |
| Tubocurarine Chloride | 2.5-5.0 |
| Vasopressin | 2.5-4.5 |
| Vincristine Sulfate | 3.5-4.5 |
| Vidarabine Concentrate | 5.0-6.2 |
| Vinblastine Sulfate | 3.5-5 0 |
| Warfarin Sodium | 7.2-8.3 |
| Verapamil | 4.1-6.0 |

It is to be understood that the above list of drugs is for purposes of illustration and does not imply that it comprises all the drugs which may be beneficially formulated or reformulated using the claimed parenteral drug delivery system. Indeed, other medically useful compositions which are encompassed by the definition of drugs herein and which effectiveness is also improved through incorporation in the present invention by improving stability, solubility, delivery, etc. include vitamins, for example vitamin B complex, vitamin $B_{12}$, vitamin K, folic acid, etc. Additionally, enzymes such as glucagon, lipase, a-amylase, etc., and nutrients such as fats, proteins, amino acids and carbohydrates may be incorporated into the present drug delivery system. Nutritional formulations using this invention may include both water soluble and water insoluble compositions, for example, vitamins, polyunsaturated corn oil, soy oil, medium chain triglycerides, amino acids, soy protein isolate, soy lecithin, corn syrup, sucrose and other nutritional entities known to be useful when normal food intake is precluded or otherwise interfered with. Peptide compositions are among other entities that can be utilized through incorporation in the claimed invention.

The intravenous delivery of many pharmaceutical preparations have been limited to the use of singular systems, as discussed, based upon the salt of a parent compound or the solubilization of the compound by agents such as a propylene glycol and alcohol, an alkaline or acidic medium. Other known systems which function primarily as blood substitutes, however, are able to solubilize and deliver drugs infused therein to a restricted degree. U.S. Patent No. 4,343,797 discloses method of preparing a synthetic blood substitute based upon a two-phase heterogeneous physico-chemical system which provides for oxygen transport and other physiological functions inherent to whole natural blood. In U.S. Patent No. 4,439,424 a preferred synthetic blood composition comprises albumin, water, sodium chloride, urea and lecithin in the form of a two-phase coacervate system which is rendered isotonic with human blood by the addition of controlled amounts of the sodium chloride. U. S. Patent Nos. 4,558,032, 4,539,204 and 4,596,778 relate to gelatin based synthetic blood substitutes based upon a two-phase coacervate system utilizing respectively, gelatin and acacia, or two gelatins or modified fluid gelatins of different isoelectric points, and gelatin or modified gelatin and lecithin. U.S. Patent No. 4,547,490 discloses a further coacervate synthetic blood derived from an aqueous solution containing albumin, soldium chloride and lecithin to which is added a non-polar or semi-polar solvent.

These known products are capable of carrying drugs, but are primarily designed to serve as a rescitative fluid much in the manner of whole natural blood. One of the principal formulation problems successfully addressed by the aforementioned patents is to maintain the integrity of the preparation, that is, to prevent a fundamental component, the stroma-free hemoglobin, from dissolving in the circulatory system after transfusion of the preparation. The successful prevention of such dissolution preserves effective oxygen transport and, in addition, endotoxic phenomena is sharply reduced or eliminated in contrast to the known blood substitutes.

In further contrast, the fundamental problem solved by the present invention is precisely the opposite, to facilitate the solubilization of the principal active component, that is, the drug after administration such that the smooth liberation of the drug into the circulation is accomplished. In addition, the present invention is notable for its ability to solubilize known water insoluble drugs in an aqueous water based medium.

Therefore, the course of action, effect and breakdown of the drug in the present delivery system are such that precipitation and irregular release of the drug does not occur. An additional significant difference between the present composition and the known blood substitutes is that the release of the drug component in the former is represented by a straight line function or zero order rate of release or exponential or first order rate of release, whereas the release of oxygen by the latter compositions follow a sigmoid curve, as does whole natural blood. This fundamental difference arises from the fact that in blood substitutes the incorporated hemoglobin plays a critical role in the release of oxygen. A sigmoid curve demonstrated by the blood substitutes indicates the specific, primary function of said compositions: to transport and release oxygen in a manner comparable to whole blood and to restore and maintain normal oncotic pressure. In contrast, the straight line function demonstrated by the present drug delivery system indicates its far greater ability to quickly disperse drugs. In fact, the present inventive drug delivery system determines rate of release. Therefore, the capability of the drug delivery of this invention can, in some instances, be determinative in life or death situations. Koster, R. and Dunning, A., New Zealand Journal of Medicine, vol. 313, No. 18, (October 31, 1985) pp. 1105-1110.

The improved abilities of the present drug delivery system are achieved with the use of an appropriate alcohol in manufacturing an initial coacervate system from which a secondary, drug delivery coacervate based system is

0256856

derived. Furthermore, adjustment of the pH of the drug delivered by the present system immediately prior to administration is not required and, as opposed to the blood substitutes which can normally be administered intravenously, the present drug delivery system permits intravenous as well as intramuscular and subcutaneous injection.

Another example of a coacervate based system is disclosed in International application PCT/US85/00859, published November 21, 1985, relating to an oral dosage composition and method of preparation to enable the oral administration of insulin. The problems addressed by the disclosed coacervate system, however, include the need for protection of the insulin against degradation by enzymes, pH, factors such as the acid-base balance and other gastrointestinal conditions and processes and, thus, the coacervate phase water in said the coacervate phase coats each individual insulin molecule to inhibit the interaction between the insulin component and the aforementioned conditions. The present invention, instead, uses an alcohol component to prepare the system and is directed to a parenteral mode of administration as opposed to an oral one.

Liposomes are in common use as drug delivery systems. However, they are fundamentally different pharmaceutic entities than coacervate systems. Examples of liposome systems include the following U.S. Patents. U. S. Patent No. 4,356,167 discloses a liposome delivery system for medicaments, wherein the liposome is comprised of an aliphatic liquid-sterol-water lamellas. U. S. Patent No. 4,394,372 discloses a process for producing liposomes which are comprised of a bilayer membrane, one layer which is aqueous and the other layer which is organic. U. S. Patent No. 4,448,765 discloses a liposome delivery system which incorporates a polymer in the vesicle wall to stabilize the composition. Physiological components are introduced into the liposomes for therapeutic administration to human or other mammalian hosts.

Fundamental differences exist between the liposome vehicle and the concervate system. Drugs incorporated into the concervate phase of the present system yield a transparent, homogeneous, monomolecular dispersion, that is, a true solution. The drugs incorporated in this phase cannot be filtered out of the solution. In contrast, liposomes comprise a heterogenous dispersion of liposomes containing a particular drug, and do not comprise a true solution. Liposomes, including the drug incorporated therein, are perpetually suspended in their vehicle and can be separate therefrom.

Moreover, the composition of the present invention comprises a large variety of surface-active agents, that is, molecules which are non-toxic, and endogenous or exogenous to the human system including lecithin, albumin, acacia solution, gelatin, modified fluid gelatin and combinations thereof. True liposomes can be prepared only from lecithin.

The methods for preparing the subject drug delivery system and liposomes are also fundamentally different. In the present method, the solubilization and dissolution of a water insoluble drug takes place in the concervate phase of said system. In contrast, the incorporation of a drug into a liposome drug delivery system results by collapsing the lecithin film around the drug. As a further difference between coacervate systems and liposomes, the exact quantity and location of a drug incorporated into a concervate system are known. When liposomes are used to incorporate and deliver drugs, an unknown quantity of the total dose of the drug is incorporated within the liposome and an unknown quantity of the drug component adheres to the external surface of the lecithin film.

Liposomes neither demonstrate the in vivo stability of the inventive composition, and are known to open up or unwrap after introduction into the body. As stated, liposomes are generally understood to consist of concentric rings comprising individual particle entities. Coacervates form solvent liquid thermodynamically stable solution phases. Liposomes cannot form such solutions. Preparation of a coacervate system using lecithin as one of its components requires the precise adjustment of concentration and conditions. In contrast, the singular step of placing 30 to 50% weight volume of lecithin in water will spontaneously produce a liposome.

According to one aspect of the present invention, there is provided a parenterally administrable composition characterised in that it comprises a non-toxic two phase liquid aqueous coacervate system, one phase of which is colloid rich, semipolar to nonpolar in character and capable of solubilizing oil soluble and water insoluble compositions of matter; and the second phase of the coacervate system is colloid poor, semipolar to polar in character and capable of solubilizing water soluble and, to a lesser degree, water insoluble compositions of matter; said colloid rich phase being insoluble and in equilibrium with said colloid poor phase; said colloid rich phase of said two phase liquid aqueous system being adapted to solubilize a medically useful composition to render it suitable for parenteral modes of administration.

According to a second aspect, the above composition may be used in the manufacture of a medicament.

According to another aspect of the invention, there is provided a method of preparing a parenterally administrable composition characterised in that it comprises the steps of: (a) dissolving a phospholipid in water and adding thereto such quantity of alcohol as will cause the phospholipid-water solution to separate into three immiscible phases; (b) separating the middle phase from the other two phases; (c) adding a surface active protein to this middle phase and storing the resulting solution, preferably under refrigeration, until said solution separates into two phases, one of which is a relatively semipolar to nonpolar colloid rich phase and, the other of which is a relatively semipolar to polar colloid poor phase; said colloid rich phase being adapted to receive a pharmacologically effective amount of a medicament.

The present invention constitutes a significant advance over the state of the art, enabling the formulation and delivery of solutions of water insoluble and water soluble parenteral drugs in an aqueous water based vehicle at the pH of body tissue. The formulations are stable, pharmacologically active and will not precipitate in body tissue or fluid. Said formulations are free of the distorted pharmacokinetics of known parental preparations, reducing or eliminating the drug-vehicle related pain and tissue damage and overcoming the problem of solubilization.

The present invention provides for a drug delivery system useful for the solubilization and delivery of pharmacologically active substances as parenterally administrated drugs at the pH of body tissue. Said system is derived from a non-toxic two phase liquid aqueous coacervate system, one phase of which is colloid rich, semipolar to nonpolar in character and capable of solubilizing oil soluble and water insoluble compositions of matter; and the second phase of the coacervate system is colloid poor, semipolar to polar in character and capable of solubilizing water soluble and, to a lesser degree, water insoluble compositions of matter; said colloid rich phase being insoluble and in equilibrium with said colloid poor phase; and wherein a drug incorporated into the colloid rich phase of said two phase liquid aqueous system yields a medically useful composition suitable for parenteral modes of administration. The component ingredients which provide for the coacervate-based drug delivery system are present in both phases, however, analytic techniques demonstrate that the colloid rich phase will have a greater concentration of said ingredients than the colloid poor phase. This higher concentration may range from 20% to 60%. This two-phase system provides a vehicle that directly solublizes the drug compositions incorporated therein thus enabling the injection at a pH of from 7.3 to 7.4, or any physiologically acceptable pH.

A preferred method to prepare the improved drug delivery system comprises the steps of: (a) dissolving a phospholipid in water and adding thereto an alcohol in an amount which will cause the phospholipid-water solution to separate into three immiscible phases; b) separating the middle phase from the other two phases; c) adding a surface active protein to this middle phase and storing the resulting solution, preferably under refrigeration, until said solution separates into two phases, one of which is a relatively semipolar to nonpolar colloid rich phase and one of which is a relatively semipolar to polar colloid poor phase; adding a water insoluble drug thereof to the colloid rich phase of the two phase liquid system in such quantity as will produce a parenteral solution containing the drug component in the desired dosage. The colloid poor phase may be used to solubilize water soluble compositions. Additionally, the drug delivery system can be prepared in the form of microencapsulated sustained release formulations (emulsions) of parenteral drugs. The microencapsulated particles range in size from less than 100 nanometers to 3 microns. In the practice of this aspect of the invention, the final product may be composed of particles of the same or different sizes.

A critical distinction between the coacervate based blood substitutes and the present invention is the use of an appropriate alcohol which is added to a first component to form a three phase system, the middle coacervate phase being then separated out and to which the second component is added. A second coacervate system results which provides the basis for the claimed drug delivery system.

In the practice of this invention, any appropriate non-toxic coacervate system can be used in the manufacture of the drug delivery system. The coacervate system incorporates non-toxic endogenous or exogenous surface-active agents, derivatives thereof and/or combinations of surface-active agents or derivatives.

One preferred coacervate system includes a phospholipid such as lecithin, a protein with surface-active properties such as albumin, an alcohol such as n-butyl alcohol, and water.

Parenteral drugs which are prepared and administered at an excessive pH, parenteral drugs that require pH adjustment immediately prior to injection and any salt of a parent drug compound which has been neutralized by means of the proper acid-base reaction to yield the parent compound may be used as the drug component in the practice of this invention.

It should be noted that while this invention solubilizes parenteral drugs as described, some dissolution of the drug also occurs in the use of this invention.

Either the colloid rich phase of the disclosed two phase liquid aqueous (coacervate) system or a combination of the colloid rich and the colloid poor phases of said system may be used to produce the finished product of this invention. The use of the colloid rich phase is preferred. Under certain conditions, the colloid poor phase can be used as in instances wherein formulations of polar and semi-polar drugs may be used to advantage. Administration of the formulations of this invention may be intravenous, intramuscular or sub-cutaneous as required.

Drug compositions commonly understood to be water insoluble, through the use of the present drug delivery system, can now be solubilized in an aqueous media. Further, the disclosed invention provides a basis for improving the manufacture and efficacy of literally hundreds of parenteral drugs.

In order to explain the invention more fully, the following is a description of a preferred method of preparing the claimed compositions.

All component ingredients are prepared, combined and/or mixed under sterile conditions. The water used in manufacturing the coacervate system must be sterile and pyrogen-free.

A phospholipid, such as lecithin, is one component in this invention. It may be derived from any suitable source. Egg lecithin is preferred. Other phospholipids such as cephalin, isolecithin, sphingomyelin, phosphatidyl serine, phosphatidic acid, phosphatidyl inositol and phosphatidyl choline, and combinations thereof, may be used in place of lecithin.

A suitable surface-active protein such as albumin comprises another ingredient. Albumin derived from any acceptable source, such as human, is acceptable. Other suitable surfactants include alpha-, beta- and gamma-globulins, gelatin, modified fluid gelatin, lipoproteins, polypeptides and mucopolysaccarides.

Alternatively, a combination of components entirely exogenous to the human system may be used to provide for the coacervate system including gelatin and acacia, or two gelatins or two modified fluid gelatins having different isoelectric points.

A suitable co-solvent may also be used in the formulation of this invention.

A preferred two-phase liquid aqueous (coacervate) system of this invention is prepared using the following steps. To 100 mls of sterile, pyrogen-free water are added 1-6% w/v of lecithin, derived from egg or other acceptable sources. The mixture is mixed or shaked vigorously until the lecithin is thoroughly dissolved. 6 to 12 mls of any acceptable alcohol such as ethyl alcohol, n-butyl alcohol, etc. is then added to the lecithin solution, and said solution is then shaken vigorously for from 10 to 30 seconds and set aside, undisturbed, for from 1 to 4 hours. Within this period of time, the solution will separate into three phases. If at the end of two hours the phases have not separated out, n-butyl alcohol may be added dropwise until the separation of phases occurs. If desired, the period of undisturbed storage may extend to 24 hours or longer. Longer periods of storage typically result in greater yields of the coacervate phase. At the close of the storage period, it will be observed that three layers have formed. The uppermost phase comprised of n-butyl alcohol is separated from the other phases and set aside since it is now extraneous to the coacervate system. The middle layer comprises approximately 50 mls of a colloid rich (coacervate) phase and the bottom layer comprises approximately 50 mls of a colloid poor (equilibrium water) phase. The proportions of the respective phases may vary from 0.5% to 99.5% by volume.

After separation of the phases is completed, from 1 to 2 grams of human serum albumin is dissolved in the colloid rich (coacervate) phase of the solution. The preparation is then stored, preferably under refrigeration, until visual inspection indicates that the preparation has separated into two phases. The upper layer comprises the colloid rich phase of the newly prepared coacervate system; the bottom layer comprises the colloid poor phase of said system. The two phases are separated, following which that quantity of drug is dissolved in the colloid rich phase as will produce an injectable product containing the standard dose or if desired, a greater or lesser quantity of drug to produce a modified clinical dose. Additionally, if desired, urea in an amount that may range from 1% to 40% w/v may be added to the coacervate phase, prior to the addition of the drug component.

With the exception of the incorporation of the optional addition of urea, the formulation as given above can be used to prepare a medically useful parenteral nutritional composition. In preparing such composition, the colloid poor and the colloid rich phases are used. Water soluble ingredients such as sucrose, corn syrup are added to the colloid poor phase in nutritionally indicated quantities while water insoluble ingredients such as soy lecithin, polyunsaturated corn oil, medium chain triglycerides, soy protein, etc. are added in nutritionally indicated quantities to the colloid rich phase. Both phases, containing their respective nutritionally indicated components, are then combined and processed to produce a microemulsion suitable for parenteral administration via intravenous drip or other acceptable means. The nutritional formulation permits the use of other sources of protein, carbohydrates and fats through the preferred ingredients are those given above. If desired, any or all of the vitamins A, B complex, C, D, E may be added to this formulation in the manner described above. Water soluble vitamins are added to the colloid poor phase; water insoluble vitamins are added to the colloid rich phase.

Drug compounds dissolved in any non-toxic glycol, such as propylene glycol, and/or alcohol may also be used in the practice of this invention.

The procedure and quantity of drug incorporated when using such compositions are the same as that described immediately above.

A partial list of drug doses to illustrate standard doses follows.

| | |
|---|---|
| Cimetidine HCl | 150 mg/ml |
| Diazepam | 5 mg/ml |
| 5-Fluorouracil | 500 mg/10ml |
| Erythromycin Lactobionate | 1 mg/ml |
| Flosuridine | 500 mg/5ml |
| Amthoteracin D | 0.1 mg/ml |
| Fluphenazine HCl | 2.5 mg/ml |
| Heparin Sodium | 1,00-20,000 units/ml |
| Haloperidol lactate | 5 mg/ml |
| Ketamine HCl | 10 mg/ml |
| Labetol HCl | 5 mg/ml |
| Lipocaine HCl | 10 mg/ml |
| Miconozole | 10 mg/ml |
| Morphine Sulfate | 0.5-1.0 mg/ml |
| Dropendal | 2.5 mg/ml |
| Imipramine HCl | 25 mg/2ml |
| Phenytoin | 100 mg/ml |
| Pentobarbital Sodium | 50 mg/ml |
| Tetracycline HCl | 250 mg/100ml |
| Thiopental Sodium | 0.2 mg/2ml |
| Verapamil HCl | 2.5 mg/ml |
| Vincristine Sulfate | 1.0 mg/ml |
| Fentanyl citrate | 0.05 mg/ml |
| Methylprednisolone Sodium Succinate | 40 mg/ml |

Once the drug is solubilized in the colloid rich phase of this invention and adjustments to the pH, if necessary, to 7.3-7.4 are made using either hydrochloric acid or sodium bicarbonate, the preparation may be administered or stored in the standard or desired dose in appropriate containers such ampules, vials etc. Alternatively, following solubilization of the drug in the coacervate phase, the previously separated colloid poor phase can be added and the resulting mixture emulsified using techniques known to produce microemulsions. Following adjustment of the pH to 7.3-7.4 by adding either HCl or sodium bicarbonate as required, the preparation is now ready for use or storage. In some instances, as noted previously, the colloid poor phase may be used to solubilize and prepare formulations of polar and semi-polar drug compositions.

This invention provides for the optional manufacture of a time-release microencapsulated preparation of the claimed composition. To prepare a microencapsulated sustained (time) release formulation (suspension) of the claimed drug delivery system, either a chemical approach using any non-toxic member of the aldehyde group, such as gluteraldehyde, or a heating process can be used. In this invention, the heating process is preferred. In this process the composition is heated at $70^{\circ}C$ for from 10 seconds to 2 minutes. The effect of the heating step is a hardening of the surface of the emulsified droplets containing the drug entity. By varying the variables of time and temperature, it is possible to obtain microemulsified droplets with a spectrum of surface hardnesses. The range of surface hardness of the microencapsulated droplets of this invention can range from fluid-like to semi-solid, i.e., gel-like to rigid. When the desired degree of surface hardness has been achieved, the droplets are filtered from the emulsion, washed thoroughly with saline or other suitable solution and then dried by any of the conventional methods. The microencapsulated droplets are then incorporated in either saline solution, the equilibrium water phase of this invention or the coacervate phase of this invention in such quantity as will result in the appropriate dose in the finished product. Differing proportions of microencapsulated droplets with differing degrees of surface hardness may be combined if desired, during the process of reconstituting the preparation using the compositions referred to immediately above. Alternatively, microemulsified droplets of the same degree of surface hardness may be used in preparing the claimed parenteral compositions. On completion of the steps described above, the composition may be administered by intravenous, intramuscular or subcutaneous injection, or stored in suitable containers.

An alternative to the above method uses virtually the same components. In the alternative method, an alcohol such as isopropyl alcohol is added dropwise to a 4% w/v solution of albumin until a two-phase liquid aqueous system is produced. The two phases are separated. The upper phase is referred to as the colloid rich phase; the lower phase known as the colloid poor phase is discarded or stored for later use to prepare additional quantities of the coacervate system. A 4% w/v quantity of lecithin powder is dissolved in the colloid rich phase creating an albumin-lecithin based colloid rich phase. The parenteral drug to be used is dissolved in the said colloid rich phase. The procedures described in connection with a preferred method can be followed to produce a preferred composition. The finished product of this invention can also be prepared either as an emulsion or a suspension when using the herein described alternate method.

## EXPERIMENTS AND SPECIFIC EXAMPLES

The following are examples of parenteral drugs compositions incorporated into the standard saline solution and into the present drug delivery system for injection. The comparative results of the parenteral preparations when dissolved in a solution and the claimed invention at a pH of 7.4 are set forth in Table 1 which follows:

### EXPERIMENT 1 10 ml of a commercial parenteral preparation of Diazepam (pH 6.5) were added dropwise to 5cc of a standard saline solution, the pH of which had been adjusted to 7.4. The results are given in Table 1 which follows.

### EXPERIMENT 2 10ml of a commercial parenteral preparation of Diazepam (pH 6.5) were added dropwise to a glass container containing 10 ml of the colloid rich phase (pH: 7) of the disclosed invention. The results are given in Table 1 which follows.

### EXPERIMENT 3 50 mgs of a commercial parenteral preparation of phenytoin (pH 12.0) were added dropwise to 1 ml of a standard saline solution, pH adjusted to 7.4. The results are given in the Table 1 which follows.

### EXPERIMENT 4 50 mgs of a commercial parenteral preparation of phenytoin (pH 12.9) were added dropwise to 1 ml of the colloid rich phase of this invention (pH 7.4). The results are given in Table 1 which follows.

## EXPERIMENT 5 100 mgs of a commercial intramuscular preparation of tetracycline HCl (pH: 2.0) were added to 5cc of a standard saline solution, pH adjusted to 7.4. The results are given in Table 1 which follows.

## EXPERIMENT 6 250 mgs of a commercial intramuscular preparation of tetracycline HCl, reconstituted as per the manufacturer's directions (pH: 2.0) were added to 5cc of the colloid rich phase of this invention (pH: 7.4.). The results are given in Table 1 which follows.

## EXPERIMENT 7 100 mgs of a commercial·intravenous preparation of tetracycline HCL (pH: 2.0) where added to 5cc of a standard saline solution, pH adjusted to 7.4. The results are given in Table 1 which follows.

## EXPERIMENT 8 250 mgs of commercial intravenous preparation of tetracycline HCL reconstituted as per the manufacturer's directions (pH: 2.0), were added to 5cc of the colloid rich phase of this invention (pH: 7.4). The results are given in Table 1 which follows.

Table 1

RESULTS OF EXPERIMENTS

COMMERCIAL PARENTERAL PREPARATIONS

|  | Diazepam (pH: 6.5) | Phenytoin (pH: 12.0) | Tetracycline HCl IM (pH: 2.0) | Tetracycline HCl IV (pH: 2.0) |
|---|---|---|---|---|
| Saline Solution (pH adjusted to 7.4) | Precipitate forms | Precipitate forms | Precipitate forms | Precipitate forms |
| Colloid rich phase of this invention (pH: 7.4) | Soluble | Soluble | Soluble | Soluble |

0256856

-24-

Examples of the methods by means of which the claimed compositions of matter may be manufactured as follows:

### EXAMPLE 1

To 100 mls of sterile, pyrogen-free water is added 4% w/v of egg lecithin. The mixture is stirred vigorously until the lecithin is thoroughly dissolved. 10 to 12 mls of n-butyl alcohol are then added to the lecithin solution, which is then shaken vigorously for from 1 to 2 hours. At the close of the storage period, it is observed that three phases have formed. The middle phase which comprises the colloid rich phase is separated from the other phases by means of a separatory funnel. 1 to 3 grams of human serum albumin is then dissolved in this phase. The solution is then stored at from 4 to 10° C until visual inspection indicates that the solution has separated into two phases and that no increase in the volume of said phases is occurring. The two phases are then separated by means of a separatory funnel. That quantity of diazepam is added to the colloid rich phase as will result in a final concentration of 5 mg of diazepam to 1 ml of coacervate solution. After this step the colloid poor (lower) phase is mixed into the colloid rich phase. The resulting mixture is then processed by emulsifying techniques well known to those skilled in the art to produce a microemulsion. The pH of the said microemulsion is then adjusted, if necessary, to 7.3-7.4 by the dropwise additon of dilute hydrochloric acid or sodium hydroxide as indicated. The preparation can then be stored in suitable containers for use by injection.

### EXAMPLE 2

The procedure of Example 1 is followed except that prior to storage or administration, the composition is subjected to a procedure designed to produce a sustained preparation. Thus, the preparation described in Example 1 is heated at 70° C. for 60 seconds. The resulting microencapsulated droplets are separated from the emulsion, washed thoroughly with sterile water, then dried thoroughly and dispersed in any vehicle appropriate for parenteral use in that quantity as will result in a finished preparation that contains 5 mg of diazepam per 1 ml of solution. On completion of this step, the composition may be administered by injection or stored in suitable containers until needed.

EXAMPLE 3

The procedure of Example 1 is followed except that 10 mls of a commercial preparation of diazepam is used in place of diazepam. (parent compound).

EXAMPLE 4

The procedure of Example 2 is followed except that 5 mgs of diazepam is used in place of the commercial parenteral preparation of diazepam.

EXAMPLE 5

The procedure of Example 1 is followed except that 50 mgs of a commercial preparation of phenytoin is used instead of diazepam.

EXAMPLE 6

The procedure of Example 2 is used except that 50 mgs of a commercial parenteral preparation of phenytoin is used in place of diazepam.

EXAMPLE 7

The procedure of Example 1 is followed except that 50 mgs of phenytoin as the parent compound is used.

EXAMPLE 8

The procedure of Example 2 is followed except that 50 mgs of phenytoin as the parent compound is used in place of diazepam.

EXAMPLE 9

The procedure of Example 1 is followed except that 7 mls of diazepam are used.

EXAMPLE 10

The procedure of Example 1 is followed except that 100 mgs of a commercial intramuscular parenteral preparation of tetracycline HCl is used in place of diazepam.

EXAMPLE 11

The procedure of Example 2 is followed except that 100 mgs of a commercial intramuscular preparation of tetracycline HCl is used in place of diazepam.

EXAMPLE 12

The procedure of Example 1 is followed except that 100 mgs of a crystalline tetracycline is used in place of diazepam.

EXAMPLE 13

The procedure of Example 12 is followed except that 250 mgs of a commercial intravenous preparation of tetracycline HCl reconstituted as per the manufacturer's directions is used instead of diazepam.

## EXAMPLE 14

The procedure of Example 1 is followed except that preparation of the composition is completed when the diazepam is dissolved in the colloid rich phase of the coacervate system. The preparation is then ready for administration or storage.

## EXAMPLE 15

The procedure of Example 1 is followed except that preparation of the composition is completed when the phenytoin is dissolved in the colloid rich phase in place of diazepam. The preparation may then be administered or stored as required.

## EXAMPLE 16

The procedure of Example 1 is followed except that tetracycline component is dissolved in the colloid rich phase coacervate system in place of diazepam. The preparation is then ready for administration or storage.

## EXAMPLE 17

The procedure of Example 1 to the point where the colloid rich phase is separated from the colloid poor phase. Instead of using the colloid rich phase to dissolve the drug component, the colloid poor phase is used to incorporate the drug component. Thus, 5 mls of commercial trimethoprin-sulfamethazole infusion is thoroughly mixed into 125 mls of the colloid poor phase. The preparation is then ready for infusion.

-28-

## EXAMPLE 18

The procedure of Example 17 is followed except that after the drug is mixed into the colloid poor phase, 125 mls of the colloid rich phase is added and the preparation emulsified. The composition may then be infused.

## EXAMPLE 19

The procedure of Example 2 is followed except that glucagon, in any quantity ranging from 0.5 mg to 1.0 mg, is substituted for the diazepam. If desired, from 25 mgs. to 150 mgs of lactose is added to the preparation following the addition of glucagon. The results are set forth in Table 2 which follows.

## EXAMPLE 20

The procedure of Example 2 is followed except that 0.5 mg of glucagon is substituted for the diazepam. The results are set forth in Table 2 which follows.

## EXAMPLE 21

The procedure of Example 1 is followed except that 1% w/v of urea is added prior to the incorporation of the drug component, which in this Example is Methyldopate HCL instead of diazepam. The results are set forth in Table 2 which follows.

## EXAMPLE 22

The procedure of Example 1 is followed except that nutritionally indicated quantities of 1-tryptophan, 1-phenylamine, 1-lysine, 1-threonine, 1-valine, 1-methionine, 1-isoleucine and 1-leucine are substituted for the diazepam.

EXAMPLE 23

The procedure of Example 1 is following with these exceptions: instead of diazepam, nutritionally indicated quantities of sucrose and corn syrup are added to the colloid poor phase after its separation from the colloid rich phase. Polyunsaturated corn oil, soy oil, soy protein powder, soy lecithin and, if desired, medium chain triglycerides are added to the colloid rich phase. Both phases are then combined and subjected to any of the known emulsification procedures which will produce a microemulsion.

Table 2

| Composition | Water adjusted to pH of 7.4 | Coacervate pH 7.4 |
|---|---|---|
| Glucagon | Precipitate | Soluble |
| Methyldopate HCL | Precipitate | Soluble |
| Lidocaine HCL | Precipitate | Soluble |
| Vitamin K | Precipitate | Soluble |
| Vitamin A as Palmitate | Precipitate | Soluble |
| Vitamin d as Cholecalciferol | Precipitate | Soluble |
| Vitamin E as dl-alpha tocopherol acetate | Precipitate | Soluble |

CLAIMS:

1.        A parenterally administrable composition characterised in that it comprises a non-toxic two phase liquid aqueous coacervate system, one phase of which is colloid rich, semipolar to nonpolar in character and capable of solubilizing oil soluble and water insoluble compositions of matter;  and the second phase of the coacervate system is colloid poor, semipolar to polar in character and capable of solubilizing water soluble and, to a lesser degree, water insoluble compositions of matter;  said colloid rich phase being insoluble and in equilibrium with said colloid poor phase;  said colloid rich phase of said two phase liquid aqueous system being adapted to solubilize a medically useful composition to render it suitable for parenteral modes of administration.

2.        A composition as claimed in claim 1, characterised in that the colloid rich phase comprises from 0.5% to 99.5% by volume and the colloid poor phase comprises from 99.5% to 0.5% by volume of the said two phase liquid aqueous system.

3.        A composition as claimed in either claim 1 or claim 2, characterised in that the two liquid phases comprise water, a phospholipid, preferably lecithin, a surface-active protein, preferably albumin, and a non-toxic alcohol, preferably n-butanol.

4.        A composition as claimed in any one of the preceding claims, characterised in that it includes a pharmacologically effective amount of a drug component including a non-toxic co-solvent, preferably propylene glycol or alcohol.

5. A composition as claimed in any one of the preceding claims, characterised in that the two phase coacervate system is separated into its component parts; a colloid rich phase into which water insoluble drugs are incorporated and a colloid poor phase into which water soluble drugs are incorporated, either single phase being suitable for parenteral administration.

6. A composition as claimed in any one of the preceding claims, characterised in that it is formulated as a sustained release preparation.

7. A composition as claimed in any one of claims 1-5, characterised in that it is formulated as a prompt release preparation.

8. The use of a composition as claimed in any one of the preceding claims, for the manufacture of a medicament.

9. A method of preparing a parenterally administrable composition characterised in that it comprises the steps of: (a) dissolving a phospholipid in water and adding thereto such quantity of alcohol as will cause the phospholipid-water solution to separate into three immiscible phases; (b) separating the middle phase from the other two phases; (c) adding a surface active protein to this middle phase and storing the resulting solution, preferably under refrigeration, until said solution separates into two phases, one of which is a relatively semipolar to nonpolar colloid rich phase and, the other of which is a relatively semipolar to polar colloid poor phase; said colloid rich phase being adapted to receive a pharmacologically effective amount of a medicament.

10.     A method as claimed in claim 9, characterised by the further step of adding a drug to the colloid rich phase of said two phase liquid system in such quantity as will produce a parenteral solution containing the drug component in the desired dosage.

11.     A method as claimed in claim 10, characterised in that a cosolvent, preferably propylene glycol or alcohol, is added to the two phase system along with said drug component.

12.     · A method as claimed in either claim 10 or claim 11, characterised by the further step, after the solubilization of the drug in the colloid rich phase, of emulsifying the two phases.